(19) Europäisches Patentamt
European Patent Office
Office européen des brevets

(11) Publication number: **0 288 907**
**A2**

(12) **EUROPEAN PATENT APPLICATION**

(21) Application number: **88106393.7**

(51) Int. Cl.4: **A61K 31/675**

(22) Date of filing: **21.04.88**

(30) Priority: **27.04.87 US 43127**

(43) Date of publication of application:
**02.11.88 Bulletin 88/44**

(84) Designated Contracting States:
**AT BE CH DE ES FR GB GR IT LI LU NL SE**

(71) Applicant: **E.R. Squibb & Sons, Inc.**
**Lawrenceville-Princeton Road**
**Princeton, N.J. 08540(US)**

(72) Inventor: **Sudilovsky, Abraham**
**712 Winchester Avenue**
**Lawrenceville New Jersey(US)**

(74) Representative: **Vossius & Partner**
**Siebertstrasse 4 P.O. Box 86 07 67**
**D-8000 München 86(DE)**

(54) **Method for inhibiting loss of cognitive functions employing an ACE inhibitor.**

(57) A method is provided for inhibiting loss of cognitive function, including memory, which may or may not be associated with Alzheimer's disease, in a mammalian species by using an ACE inhibitor, which is a phosphonate substituted amino or imino acid or salt, such as SQ 29,852 for the preparation of a drug which is to be administered over a prolonged period of treatment.

EP 0 288 907 A2

## METHOD FOR INHIBITING LOSS OF COGNITIVE FUNCTIONS EMPLOYING AN ACE INHIBITOR

The present invention relates to a method for inhibiting loss of cognitive functions, including memory, which are associated with different types of dementias in mammalian species by using an ACE inhibitor, which is a phosphonate substituted amino or imino acid or salt, such as SQ 29,852 for the preparation of a pharmaceutical drug which is to be administered over a prolonged period of time.

In accordance with the present invention, a method is provided for the preparation of drugs for inhibiting loss of cognitive functions such as memory, attention span, concentration and ability to learn or for treating or delaying progression of Alzheimer's disease or other types of dementias, in mammalian species over a prolonged period. The pharmaceutical drugs comprise a therapeutically effective amount of an angiotensin converting enzyme inhibitor which is a phosphonate substituted amino or imino acid or salt thereof, and they are systemically, such as orally or parenterally, administered over a prolonged period, to inhibit loss of cognitive function during such period.

The invention is useful in treating or delaying progression of primary degenerative dementias arising in the senium and presenium such as Alzheimer's disease, Pick's disease and Binswanger's disease, and vascular dementias such as arterialsclerotic dementias including multiple infarct dementia and Binswanger's disease.

The angiotensin converting enzyme inhibitor which may be employed herein includes any of the phosphonate substituted amino or imino acids or salts disclosed in U. S. Patent No. 4,452,790 with (S)-1-[6-amino-2-[[hydroxy-(4-phenylbutyl)phosphinyl]oxy]-1-oxohexyl]-L-proline (SQ 29,852) being preferred.

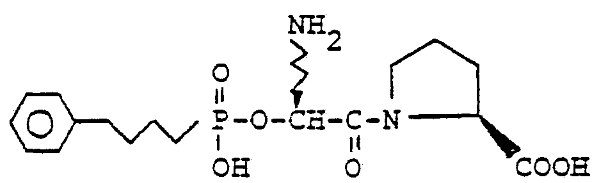

SQ 29,852

U. S. Patent No. 4,452,790 to Karanewsky et al is directed to angiotensin converting enzyme inhibitors which are phosphonate substituted amino or imino acids and salts thereof having the formula

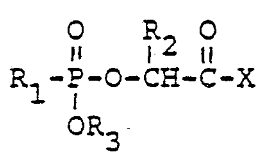

wherein X is an imino or amino acid of the formula

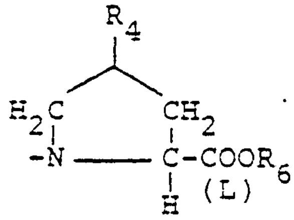 , 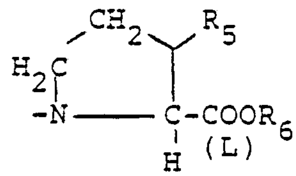 ,

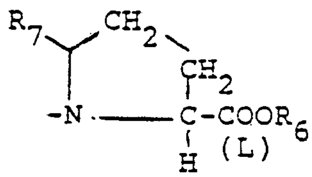 , 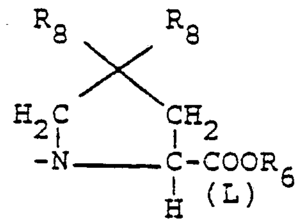 ,

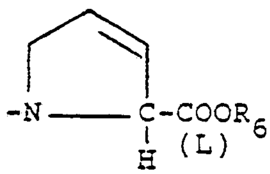 , 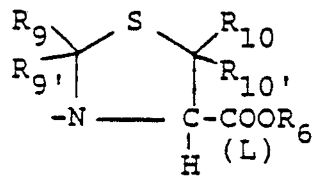 ,

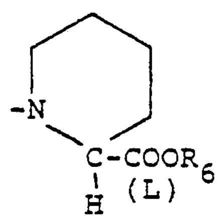 , 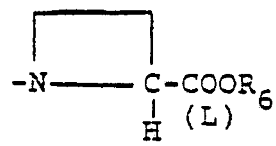 , or

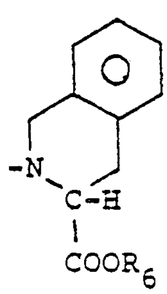 , 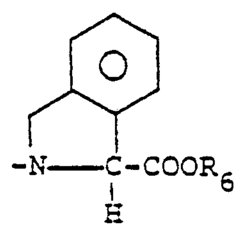 , 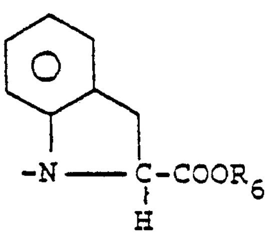

or 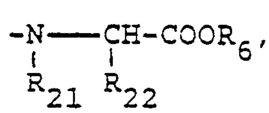

$R_4$ is hydrogen, lower alkyl, halogen, keto, hydroxy, $-NH-\overset{O}{\overset{\|}{C}}-$lower alkyl, azido, amino, 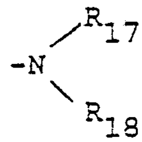 ,

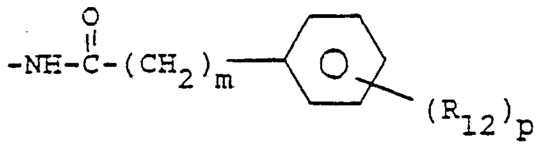 ,

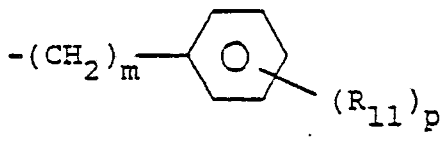 , 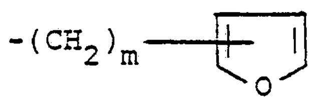 ,

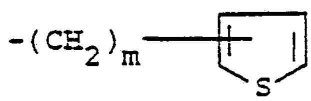 , 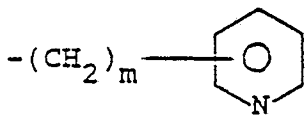 ,

a 1- or 2-naphthyl of the formula

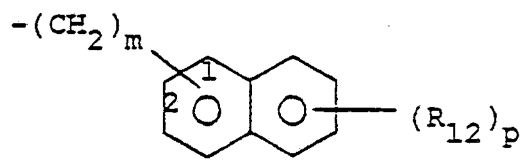 ,

$-(CH_2)_m-$cycloalkyl, 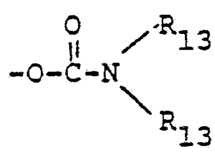 , -O-lower alkyl,

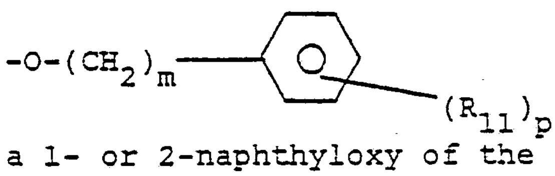 ,

a 1- or 2-naphthyloxy of the formula

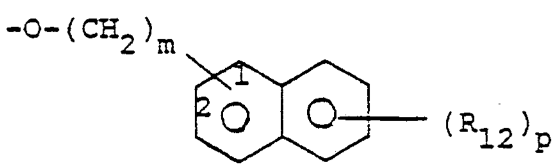 ,

-S-lower alkyl,

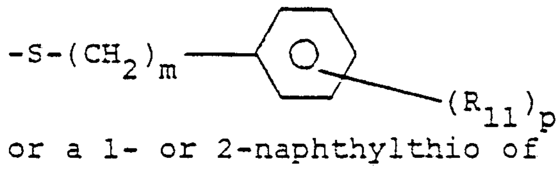 ,

or a 1- or 2-naphthylthio of the formula

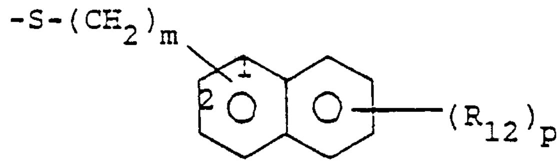 ,

$R_5$ is keto, halogen, 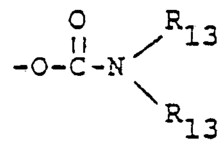 ,

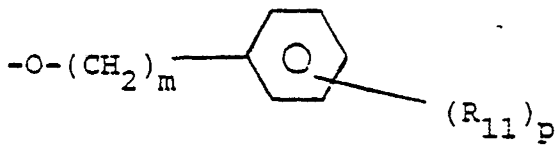 ,

-O-lower alkyl, a 1- or 2-naphthyloxy of the formula

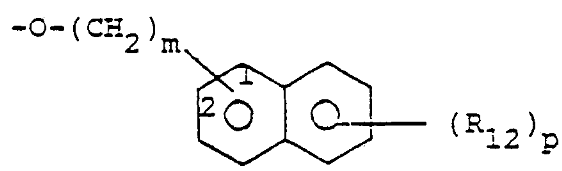 ,

-S-lower alkyl,

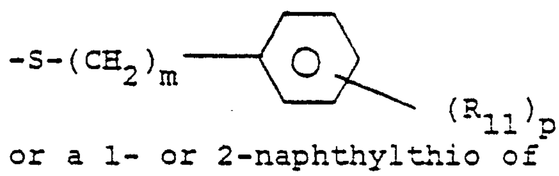 ,

or a 1- or 2-naphthylthio of the formula

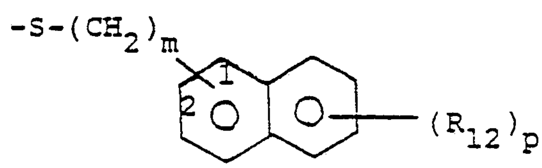 ,

$R_7$ is keto or

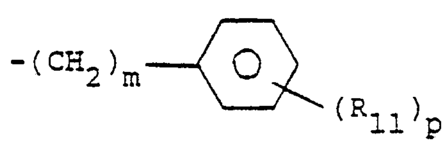 ,

Each $R_8$ is independently halogen or $-Y-R_{14}$.

$R_9$, $R_9'$, $R_{12}$ and $R_{10}'$ are independently selected from hydrogen and lower alkyl or $R_9'$ $R_{10}$ and $R_{10}'$ are hydrogen and $R_9$ is

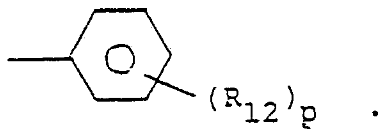

$R_{11}$ is hydrogen, lower alkyl of 1 to 4 carbons, lower alkoxy of 1 to 4 carbons, lower alkylthio of 1 to 4 carbons, chloro, bromo, fluoro, trifluoromethyl, hydroxy, phenyl, phenoxy, phenylthio, or phenylmethyl.

$R_{12}$ is hydrogen, lower alkyl of 1 to 4 carbons, lower alkoxy of 1 to 4 carbons, lower alkylthio of 1 to 4 carbons, chloro, bromo, fluoro, trifluoromethyl or hydroxy.

m is zero, one, two or three.

p is one, two or three provided that p is more than one only if $R_{11}$ or $R_{12}$ is hydrogen, methyl, methoxy, chloro or fluoro.

$R_{13}$ is hydrogen or lower alkyl of 1 to 4 carbons.

Y is oxygen or sulfur.

$R_{14}$ is lower alkyl of 1 to 4 carbons,

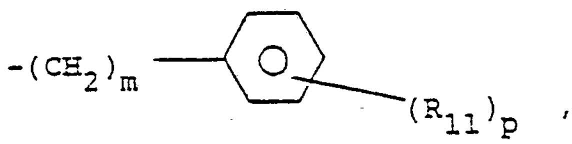

or the $R_{14}$ groups join to complete an unsubstituted 5-or 6-membered ring or said ring in which one or more of the carbons has a lower alkyl of 1 to 4 carbons or a di(lower alkyl of 1 to 4 carbons) substituent.

$R_{21}$ is hydrogen, lower alkyl, cycloalkyl, phenyl or

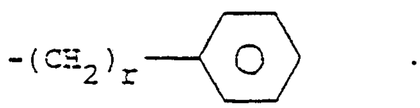

$R_{22}$ is hydrogen, lower alkyl,

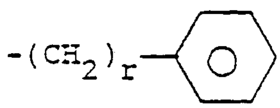 , 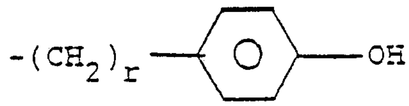 ,

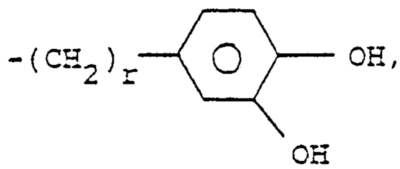 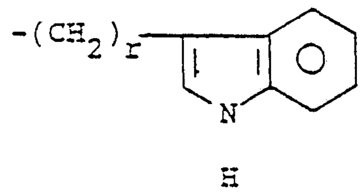 ,

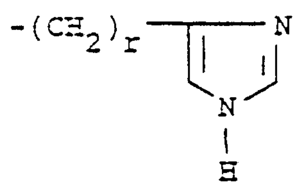 , $-(CH_2)_r-NH_2$, $-(CH_2)_r-SH$,

$-(CH_2)_r-S-$ lower alkyl, 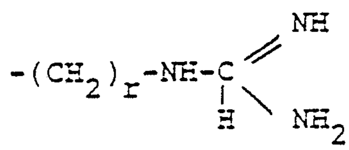 ,

or 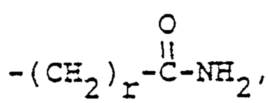 ,

r is an integer from 1 to 4.

$R_1$ is alkyl of 1 to 10 carbons, aminoalkyl, haloalkyl,

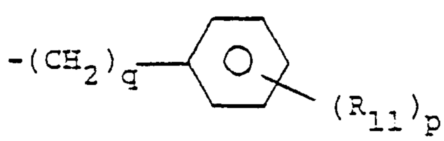 , $-(CH_2)_q$-cycloalkyl,

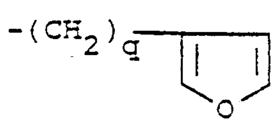 , 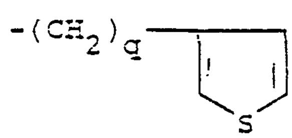 ,

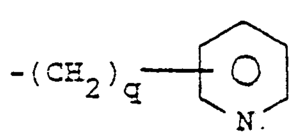 , or 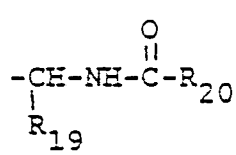

wherein q is zero or an integer from 1 to 7 and $R_{12}$ and p are as defined above.

$R_{19}$ and $R_{20}$ are independently selected from hydrogen, lower alkyl, halo substituted lower alkyl,

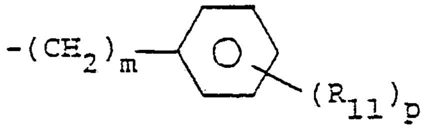 , $-(CH_2)_m$-cycloalkyl,

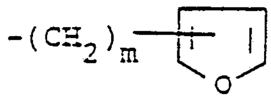 , 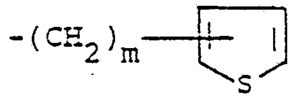 ,

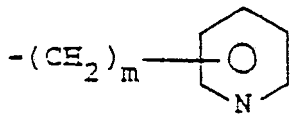 , or 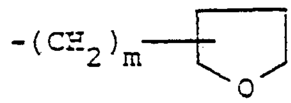

wherein m, $R_{11}$, and p are as defined above.

$R_2$ is hydrogen, lower alkyl, halo substituted lower alkyl,

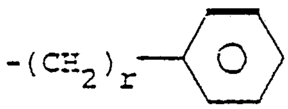 , 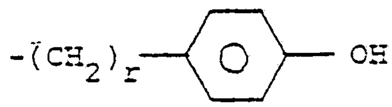 ,

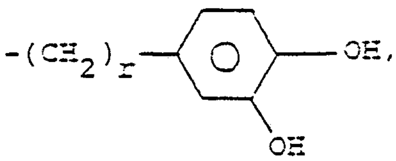 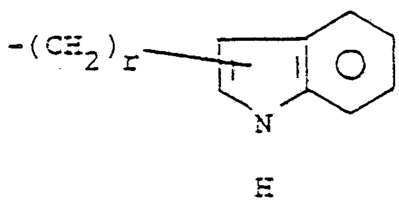

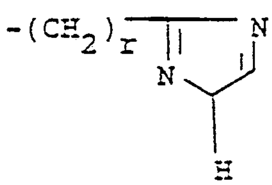 , $-(CH_2)_r-NH_2$, $-(CH_2)_r-SH$,

$-(CH_2)_r$-S- lower alkyl, 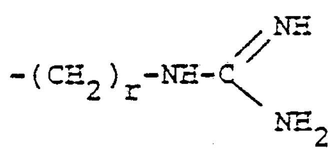 or

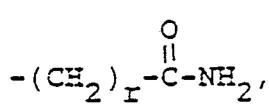

wherein r is as defined above.

$R_3$ and $R_6$ are independently selected from hydrogen, lower alkyl, benzyl, alkali metal such as Li, Na or K, benzhydryl, or

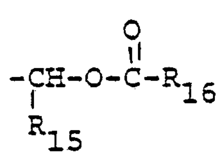

wherein $R_{15}$ is hydrogen, lower alkyl, cycloalkyl or phenyl, and $R_{16}$ is hydrogen, lower alkyl, lower alkoxy, phenyl, or $R_{15}$ and $R_{16}$ taken together are $-(CH_2)_2-$, $-(CH_2)_3$ $-CH=CH-$, or

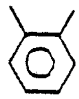 .

$R_{17}$ is lower alkyl, benzyl, or phenethyl.

$R_{18}$ is hydrogen, lower alkyl, benzyl or phenethyl.

The disclosure of the above-mentioned patent is incorporated herein by reference.

In carrying out the present invention, pharmaceutical drugs containing the angiotensin converting enzyme inhibitor as defined above may be administered to mammalian species, such as monkeys, dogs, cats, rats, humans, etc. and as such may be incorporated in a conventional systemic dosage form, such as a tablet, capsule, elixir or injectable. The above dosage forms will also include the necessary carrier material, excipient, lubricant, buffer, antibacterial, bulking agent (such as mannitol), anti-oxidants (ascorbic acid of sodium bisulfite) or the like. Oral dosage forms are preferred, although parenteral forms are quite satisfactory as well.

With regard to such systemic formulations, single or divided doses of from about 0.1 to about 500 mg, preferably from about l to 100 mg,one to four times daily, may be administered in systemic dosage forms as described above for a prolonged period, that is, for as long as inhibition of loss of cognitive function is to continue. Sustained release forms of such formulations which may provide such amounts biweekly, weekly, monthly and the like may also be employed. A dosing period of at least one week is required to achieve minimal benefit.

The accompanying Figure is a graph of mean percent of shuttle avoidance responding in male rats one hour after intraperitoneal administration of SQ 29,852 at l mg/kg, 10 mg/kg and 30 mg/kg and saline versus days of treatment.

The following Examples represent preferred embodiments of the present invention.

Example 1

An SQ 29,852 formulation suitable for oral administration in inhibiting loss of cognitive functions is set out below.

1000 tablets each containing 100 mg of (S)-1-[6-amino-2-[[hydroxy(4-phenylbutyl)phosphinyl]oxy-1-oxohexyl]-L-proline were produced from the following ingredients.

(S)-1-[6-Amino-2-[[hydroxy(4-phenylbutyl)phosphinyl]oxy-1-oxohexyl]-L-proline (SQ 29,852) 100 g
Corn starch 50 g
Gelatin 7.5
Avicel (microcrystalline cellulose) 25 g
Magnesium stearate 2.5 g

The SQ29,852 and corn starch are admixed with an aqueous solution of the gelatin. The mixture is dried and ground to a fine powder. The Avicel and then the magnesium stearate are admixed with the granulation. This is then compressed in a tablet to form 1000 tablets each containing 100 mg of active ingredient which is used for inhibiting loss of cognitive functions.

Example 2

By substituting 100 g of the ACE inhibitor (±)-l-[2-[[hydroxy(4-phenylbutyl)phosphinyl]oxy]-1-oxopropyl]-L-proline, dilithium salt for the SQ 29,852 in Example 1, 1000 tablets each containing 100 mg of such ACE inhibitor are produced which is useful in inhibiting loss of cognitive functions.

Example 3

1000 tablets each containing 200 mg of I are produced from the following ingredients:

1-[(S)-2-[[[(±)-1-Benzoylamino)-3-phenylpropyl]phosphinyl]oxy]-1-oxopropyl]-L-proline, dilithium salt (ACE inhibitor) 200 g
Lactose 100 g
Avicel 150 g
Corn starch 50 g
Magnesium stearate 5 g

The ACE inhibitor, lactose and Avicel are admixed, then blended with the corn starch. Magnesium stearate is added. The dry mixture is compressed in a tablet press to form 1000 505 mg tablets each containing 200 mg of active ingredient. The tablets are coated with a solution of Methocel E 15 (methyl cellulose) including as a color a lake containing yellow #6. The resulting tablets are useful in inhibiting loss of cognitive functions.

Example 4

Two piece #1 gelatin capsules each containing 250 mg of ACE inhibitor are filled with a mixture of the following ingredients:

(±)-1-[2-[[[(2,2-Dimethyl-1-oxopropoxy) methoxy](4-phenylbutyl)phosphinyl]oxy]-1-oxopropyl]-L-proline 250 mg
Magnesium stearate 7 mg
USP lactose 193 mg.

The resulting capsules are useful in inhibiting loss of cognitive functions.

Example 5

An injectable solution for use in inhibiting loss of cognitive functions is produced as follows:

SQ 29,852 500 mg
Methyl paraben 5 mg
Propyl paraben 1 mg
Sodium chloride 25 g
Water for injection qs. 5 1.

The SQ 29,852 preservatives and sodium chloride are dissolved in 3 liters of water for injection and then the volume is brought up to 5 liters. The solution is filtered through a sterile filter and aseptically filled into presterilized vials which are then closed with presterilized rubber closures. Each vial contains 5 ml of solution in a concentration of 100 mg of active ingredient per ml of solution for injection.

Example 6

Tablets for use in inhibiting loss of cognitive functions are prepared as described in Example 1 except that (±)-1-[2-[[hydroxy(4-phenylbutyl)phosphinyl]oxy]-1-oxopropyl]-L-proline, disodium salt is used in place of SQ 29,852.

Example 7

The following experiments were carried out to demonstrate the effectiveness of the ACE inhibitor SQ 29,852 to improve cognition and cognitive impairment.

Techniques used:

a) Ability to improve basic performance and to antagonize a scopolamine impairment in a mouse habituation test.

b) Ability to improve basic performance and to antagonize a scopalamine impairment in a food reinforced alternation task in the rat using an elevated T maze.

c) Ability to improve discriminative and reverse learning in the marsoset using the Wisconsin General Test Apparatus.

a) ABILITY TO IMPROVE BASIC PERFORMANCE AND TO ANTAGONIZE A SCOPOLAMINE IMPAIRMENT IN A MOUSE HABITUATION TEST

Methods

The studies used a black:white test box procedure as described below. Male albino (BKW) mice were used, initially weighing 25-30 g. In their home cage, mice were housed in groups of 10 and given free access to food and water. The mice were kept on a 12 hour light and 12 hour dark cycle with lights off at 8:00 a.m. and on at 8:00 p.m.

The test box consisted of an open-topped box (45 × 27 × 27 cm), 40% of the area painted black and illuminated with a dim red light (1 × 60 W), the other painted white and brightly illuminated with white light (1 × 60W) located 17 cm above the box. Access between the two areas was enabled by means of a 7.5 × 7.5 cm opening located at floor level in the center of the partition (which also served to prevent diffusion of light between the two compartments of the test box). The floor area was lined into 9 cm squares.

The habituation test was carried out daily by placing mice in the center of the white section of the test box (mice taken from dark home environment in a dark container, to the experimental room maintained in low red lighting, and would normally be averse to the bright white conditions). Testing was carried out between 8:30 and 12:30 p.m. The test period was 5 minutes per day. Behavior was assessed via remote video recording, and the following measures taken:

1. Latency to move from the white to the black section (sec).

2. Numbers of exploratory rears in the white and black sections during the 5 minute test.

3. Numbers of line crossings (exploratory locomotion) in the white and black sections during the 5 minute test.

4. Time spent in the black section of the box during the 5 minute test.

5. Numbers of transitions between the black and white sections of the test box during the 5 minute test (since this parameter was not changed in any situation in the present studies, data for transitions is not given or commented on further).

Generally, as animals habituated to the test system, they would move into the black section of the box where behavioral exploration was exhibited as exploratory rears and line crossings.

Scopolamine was used at a dose of 0.25 mg/kg i.p. to disrupt habituation patterns. This could be achieved by a single acute challenge with scopolamine which disrupted the learning patterns on the day of treatment, with subsequent recovery, or by continued daily treatment with scopolamine 1 hour before test. The dose of scopolamine was carefully selected as one which did not cause autonomic disturbance (0.25 mg/kg i.p. methyl scopolamine failed to influence behavior). Under the influence of 0.25 mg/kg i.p. scopolamine mice would go to the door in the partition, investigate the opening and pass the head or body through, but without association of the dark environment with escape from the brightly-lit averse environment.

Results

The normal learning curve for mice in the habituation test was 5-6 days as evidenced by reduced rearings and line crossings in the white compartment, increased in the black, reduced latency to move to the black and increased % of time spent in the black. Acutely administered scopolamine causes impairment in control animals. Example data is given here for rears: mice had 'learned' to avoid the white averse environment and by day 6 were carrying out most of their behavior in the black - this was prevented by scopolamine which caused an impairment characterized by increased activity in the white, decreased in the black. This impairment caused by scopolamine can be prevented by arecoline. The selection of dose and route for arecoline are critical to avoid unwanted autonomic disturbance. The arecoline is given continuously by intraperitoneal infusion from Alzet osmotic minipumps at a dose of 50 mg/kg/day. It is interesting that while the continuous treatment with this dose of arecoline inhibited the scopolamine impairment of habituation, the time course of the basic 'learning' or habituation was unaffected by the presence of arecoline. This contrasts with findings for the ACE inhibitor SQ 29,582.

Using the same procedure as described above, control mice and mice treated with SQ 29,852 were subject to the habituation procedure and challenged with scopolamine on days 6 and 10. Firstly, it was seen that the basal learning procedure was speeded by treatment with SQ 29,852 at doses as low as 0.0005mg/kg b.d. secondly, the treatments with Q 29,852 were shown to completely antagonize the impairments caused by scopolamine. SQ 29,852 doses were lowered on days 7-10 to 0.00005mg/kg i.p. b.d.: this is because an anxiolytic potential was becoming apparent using the twice daily, continuous treatment regime.

Assessments of the potential of hydergine to improve cognitive function in the mouse habituation test utilized the same test protocol as described so far for arecoline and the ACE inhibitor. Hydergine was obtained as a proprietary product and the human dose titrated to mouse for single daily challenge, orally, 60 minutes before test. Treatment with hydergine was clearly shown to enhance 'learning' in the mouse habituation test. Rearing in the white section rapidly diminished as this behavior correspondingly increased in the black, and crossings in the white decreased significantly below control values by day 2 of testing, again with corresponding increases in the black and increased % of time in the black was significant on day 2 as were the reductions in latencies to move from the white to the black section on days 2, 3 and 4 of testing.

The treatment with hydergine was not associated with any anxiolytic potential and the dose regime was maintained constant at 0.1mg/kg p.o. daily. After 4 days some motor impairment and sedation developed in a small proportion of animals; this particularly influenced the latency to move from the white environment and data for such animals had to be excluded from analyses.

A very important observation was that while hydergine (like the ACE inhibitor but in contrast to arecoline) could enhance basal learning, it was not able to antagonize the influence of scopolamine to impair performance whether measured as changed rearing, changed line crossings or changed % time in black, and latency to move out of the white, aversive environment. This failure to antagonize, indeed, to any way influence the impairment caused by scopolamine contrasts with the marked antagonistic effects of arecoline and the ACE inhibitor SQ 29,852.

In a further series of experiments mice were allowed to habituate for 10 days and then were challenged daily with scopolamine, 0.25mg/kg. The habituation was impaired throughout the time of scopolamine challenge. If, after impairment with scopolamine was established, mice were given arecoline (50mg/kg/day by intraperitoneal infusion from Alzet osmotic minipumps), or SQ 29,852 (0.5µg/kg i.p. b.d.) daily with the scopolamine treatment, then the scopolamine impairment was completely prevented.

b) ABILITY TO IMPROVE BASIC PERFORMANCE AND TO ANTAGONIZE A SCOPOLAMINE IMPAIRMENT IN A FOOD REINFORCED ALTERNATION TASK IN THE RAT USING AN ELEVATED T MAZE

Methods

The studies used male Lister hooded rats initially weighing 300-350 g. Rats were normally housed in groups of 5 in a room maintained at 22±1°C, on a 12 hour light:dark cycle with lights on at 8:00 a.m. and off at 8:00 p.m. The test room was maintained under identical conditions, and was sound-proofed.

The apparatus and technique used was essentially that of Salamone et al. (Behav. Brain Res. 13, 63-70, 1984) using a T maze constructed of wood and elevated 30cm from the ground with side arms measuring 60cm × 10cm and start arm measuring 80cm × 10cm. A small metal cup was placed towards the end of each side arm; these held the reward pellets as appropriate. A line was marked 20cm from the start of each side arm.

Animals were food deprived excepting for 1 hour post-test, for 2 days prior to testing and throughout the 9 day test period, but water was available 'ad libitum'. Animals maintained 85% of normal body weight throughout testing. A few banana-flavored reward pellets were mixed with the food to habituate the rats to the taste of the pellets. Our rats showed clear preference for banana-flavored pellets as compared with their normal laboratory chow.

Rats were allowed 10 minutes habituation to the T maze on day 1 (both arms baited with banana-flavored reward pellets, 4 × 45mg pellets in each cup) and were subject to a pretraining period of reinforced alternation on days 2-5 of test, with training on days 6-9. All training consisted of paired trials (each pair constituting a 'run'), the first being 'forced' in that one arm was blocked with a wooden barrier while the other was baited (for a positive response on the forced trial the rat must take the food). The second was a 'choice' trial in which reward pellets were placed in the arm opposite to that reinforced on the first trial of the pair. A correct choice was when the rat entered the arm containing the food on the choice trial, crossing the point marked 20cm from the start of the side arm.

In addition, to correct/incorrect choice, latency to reward was recorded for both forced and choice trials. 4 runs/day were carried out on pretraining days (inter-trial interval 9 sec, inter-run interval 30 sec), 6 runs/day during training (inter-trial interval 30 sec, inter-run interval 60 sec).

6 groups of animals (n = 7 per group) were used as follows:

1. Control group - saline 1 ml/kg i.p. b.d.
2. Scopolamine group - scopolamine 0.25 mg/kg i.p. b.d.
3. Arecoline group _ arecoline 30 mg/kg/day by intraperitoneal infusion from an Alzet osmotic minipump (carefully selected as maximum dose tolerated).
4. SQ 29,852 group - SQ 29,852 1.0 mg/kg i.p. b.d.
5. Scopolamine + arecoline group.
6. Scopolamine + SQ 29,852 group.

Results

Scopolamine was shown to impair performance in the food reinforced alternation task using an elevated T maze. This impairment was seen as delayed forced latencies, delayed choice latencies and reduced % correct responses. This impairment was evident both during the pretraining days (2-5) and training days (6-9). At no time was arecoline or SQ 29,852 treatment shown to enhance basal performance in the T maze task. However, arecoline and SQ 29,852 were shown to antagonize the effects of scopolamine on days 2-5. The antagonism afforded by SQ 29,852 was less marked during days 6-9: that afforded by arecoline was more consistent.

Using the same strain of rats and the same doses of SQ 29,852 (1.0 mg/kg i.p.) assessment of anxiolytic action was carried out using an upscaled version of the black:white test box suitable for rat testing. Both compounds showed an anxiolytic profile and it is considered that this activity may have seriously interfered with responding on the T maze. Thus, while animals were challenged by a novel task on the first few days of training, this novelty was not apparent as learning progressed, and rats were noted to be 'nonchalant' to the test situation. However, the data obtained with SQ 29,852 shows that a scopolamine impairment in a food reinforced alternation task in the rat using an elevated T maze can be antagonized by S 29,852.

c) ABILITY TO IMPROVE DISCRIMINATIVE AND REVERSE LEARNING IN THE MARMOSET USING THE WISCONSIN GENERAL TEST APPARATUS

Methods

The studies used male and female marmosets aged 15-18 months (300-340 g) which had been bred at University of Bradford, England, or bought as weanlings, and had been regularly handled. All experiments were carried out by one experimenter since, while it is possible for two experimenters to work together for double-blind dosing of animals, the relationship built between the experiment/marmoset makes it impossible to design the experiments for double-blind use of marmosets. Marmosets used in the present studies were coded 081, J59 and 025.

Shaping discrimination learning and training

The procedures followed for shaping and reverse learning were standard and would be applied to any marmoset when using the WGTA.

i) Shaping and discrimination learning

The animal is first presented with open, baited food wells and the stimuli are gradually moved over the wells on successive trials until they are completely covered. When the animal will respond to both stimuli (both are rewarded at this stage) and at both food wells, shaping is complete and discrimination learning, where the reward is always put under one and the same stimulus, can begin. For stimuli one can use small toy figures or 'junk' objects about 5 cm in the largest dimension (junk objects are pen-tops, bottle tops, etc.). Animals 081, J59 and 025 were generally nervous of the small toy figures presented to them (e.g. farm animals, cowboys and indians) and so the discriminative learning used a rubber bung (positive) and syringe needle cap (negative). Animals may meet different objects during its experimental career. However, a marmoset within our limited experience (and in the wider experience of others, Baker and Ridley) never forgets the first shaping and discriminative learning trials, and the first objects used, the rubber bung and needle cap for the marmosets used in the present studies, can be used to help animals when they are later given very complex tasks, and lose confidence.

In order to ensure that the animal learns the discrimination on the basis of the stimulus association rather than because the reward is always on, say, the left, the left/right position of the stimuli is varied according to a "pseudo-random" schedule. In one such schedule, described by Gellerman (1933; J. Genet. Psychol. 42, 206-208), the reward appears unpredictably on the left or right, though in each block of 10 trials it appears 5 times on each side. This schedule was used in the present experiment. Marmosets are easily distracted by visual as well as auditory interference. The test cage therefore has opaque walls on all sides and a smoked Perspex panel facing the shutter. Sawdust should not be used on the floor of the cage otherwise the animal may spend a considerable time sifting through it.

It was not necessary to maintain food deprivation during these training sessions. The procedure was to feed the animal its normal daily diet after the day's training session (sessions generally restricted to 8:30-11:30 a.m. and 12:00-3:00 p.m., feeding at 4:00 p.m.).

(ii) Training on the Gellerman Schedule

Animals are tested usually on 4 or 5 consecutive days to gain 90/100 correct choices. They are then allowed a 1 week break before confirming a criterion of 18/20, and the 9/10. It is important that the experimenter knows the animals, and never makes them 'work' until bored or fail to concentrate. For 081, J59 and 025 the critical challenge was either 40 trials/days or 15 minutes of trials.

To prevent 'boredom' weekend breaks appeared very important and therefore generally experiments are carried out over a maximum 5 days. However, since there appeared to be a delay in onset of the action

of SQ 29,852, the test time was extended to 8 days.

SQ 29,852 was prepared in normal saline and was given twice daily (close to 7:00 a.m. and 7:00 p.m. for animals tested 12:00-3:00 p.m., 8:00 a.m. and 8:00 pm. for animals tested 8:30-11:30 a.m., but always 60 minutes before test).

iii) Reverse training

Objects used for the reverse training remained constant at yellow flask top/red pen top. Objects were presented until six correct consecutive trials were recorded, and the number of attempts before the 6 correct trials noted. Positive objects (with food reward) were alternated. For example, day 1 red pen top was first positive object, after 6 consecutive selections of the red pen top, the yellow flask top became the positive object, and was presented until it was selected on 6 consecutive attempts. The last positive object of each day (yellow flask top in this case) would be the first positive object of the following day.

On each day of testing, marmosets were subjected to a reverse learning task immediately after the discriminative task. For example, the objects used were yellow flask top/red pen top. If the yellow flask top was the last positive object of the previous day, it would be the first positive object of the test day and a marmoset would be expected to discriminate this from the red pen top by selecting the yellow flask top on 6 consecutive attempts. The number of attempts before this criterion was reached was determined on each day and the mean numbers for days 1-4 (vehicle), 5-8 (vehicle or SQ 29,852) and 9-12 (vehicle or SQ 29,852) are given (termed D for first discriminative task). Marmosets were then subject to a reverse learning task, i.e., on completion of the 6 correct selections of the yellow flask top, the marmoset would be required to reverse to selecting the red pen top on 6 consecutive occasions (this now being the positive, food rewarded object). The mean number of attempts made in this reverse situation was determined for days 1-4, 5-8 and 9-12 as above, and indicated R.

Results

During the first 4 days of the trials the three marmosets found reverse training more difficult than the initial discriminative learning (approximately 9 trials to criterion on discriminative learning, D, and 12 to criterion on reverse learning, R). Animal 081 was maintained on vehicle and he maintained responding on discriminative learning, continuing to find reverse learning difficult (on days 9-12 10 trials to criterion on discriminative learning but 18 on reverse learning).

The performance of animal J59 improved gradually over 8 days of treatment with SQ 29,852, 0.1 mg/kg s.c. b.d. (11>9>7 for discriminative learning, 13>9>7 for reverse learning), while animal 025 failed to improve during the first 4 days of treatment, but did improve over the subsequent 4 day period (8>5 for discriminative learning, 12>9 for reverse learning).

These experiments were carried out double blind. However, the experimenter made full comments on the quality of behavior of these 3 marmosets during testing, all of which were well known in terms of their normal behavioral repertoire in the Wisconsin test. During treatment with SQ 29,852, the comment was consistently made that the animals appeared "too carefree to work". The dose of SQ 29,852 used is markedly anxiolytic in the marmoset, and it is our experience that animals fail to work well when undergoing full anxiolysis. Nevertheless, the size of improvement seen in J59 and 025 as important in that these animals have shown constant responding over many months. It is clear that SQ 29,852 is able to improve performance in both discriminative and reverse learning tasks in the marmoset.

CONCLUSIONS

ASSESSMENT OF ABILITY TO IMPROVE COGNITION AND COGNITIVE IMPAIRMENT

a) A mouse habituation test was used in which mice were repeatedly placed in the white compartment of a white:black test box. On repeated exposure to the test situation, mice 'learn' to avoid the averse white,

brightly-lit environment and move rapidly into the black where they spend a larger proportion of time and show most exploratory rearings and line crossings.

The habituation (learning) time is 5-6 days. This basic 'learning' time was not influenced by arecoline (50 mg/kg/day given by continuous intraperitoneal infusion: dose and route selected to avoid unwanted autonomic effects). However, this dose of arecoline successfully antagonised an impairment in habituation caused by acute challenge with scopolamine (0.25 mg/kg i.p., dose again carefully selected to avoid excessive peripheral autonomic disturbance, and particularly to avoid influence on vision which can influence performance in the test: lack of effect on vision was established by visual observation and by measurement of pupil function). Methyl scopolamine at a dose of 0.25 mg/kg i.p. failed to influence mouse habituation. The effect of scopolamine was marked: animals which had learned to avoid the white environment failed to enter the black, excepting for short periods of time, even though they easily found the door, and thus the rapid exit into the black and avoidance of the white environment was prevented by scopolamine treatment.

In contrast to arecoline, the speed of habituation was enhanced by treatment with low doses of SQ 29,852 (0.0005 mg/kg i.p. b.d.). Anxiolytic potential, which influenced the test procedure, generally developed on longer term treatment with these doses of the ACE inhibitor, and doses for use in the habituation test were subsequently lowered. SQ 29,852 was found to antagonize the impairment in habituation performance caused by acute challenge with scopolamine. The dose which was fully effective against scopolamine was 0.0005 mg/kg i.p. b.d. SQ 29,852.

Hydergine (0.1 mg/kg p.o. once daily) was shown to speed the habituation process in a similar manner to the SQ 29,852 ACE inhibitor (both in contrast to the failure of arecoline), but hydergine treatment failed to influence the impairment in habituation caused by acute challenge with scopolamine (which contrasts with the actions of both the SQ 29,852 ACE inhibitor and the cholinomimetic agent).

Further studies allowed habituation to progress for 10 days before continuous impairment by scopolamine given daily for up to 14 days. This persistent impairment caused by scopolamine could be antagonized by arecoline (50 mg/kg/day by intraperitoneal infusion) and by SQ 29,852 (0.5 μg/kg i.p. b.d.).

b) In a food reinforced alternation task in the rat using an elevated T maze arecoline (30 mg/kg/day by intraperitoneal infusion) and SQ 29,852 (1.0 mg/kg i.p. b.d.) were shown to antagonize an impairment caused by scopolamine. This antagonism was particularly marked on pretraining days 2-5, but was less marked on training days 6-9. This may reflect the fact that the test becomes less challenging for the rats as training progresses, and that SQ 29,852 is anxiolytic at the dose used. Thus, it was observed that rats became increasingly 'nonchalant' about working on the test paradigm. Whatever, the data obtained with arecoline and SQ 29,852 clearly indicates that a scopolamine impairment in learning in a rat test can be improved both by a cholinomimetic and SQ 29,852.

c) Marmosets were trained in a Wisconsin General Test Apparatus to discriminate between food rewarded and non-rewarded objects. After shaping to a criterion of 90/100, 18/20 and 9/10 correct reponses (the 9/10 being repeated on several days), objects were changed and marmosets expected to select a new positive object on 6 consecutive occasions (discriminative learning), and then to change to selecting the second object (now positive in terms of food reward) on 6 consecutive occasions (reverse learning). Marmosets used had shown constant responding over many months and there had been no improvement in either discriminative or reverse learning. However, after 8 days of treatment with 0.1 mg/kg s.c. b.d. SQ 29,852 marmosets showed clear improvement in both discriminative and reverse learning. The onset of action appeared to be delayed for some 4 days. It was also considered that the anxiolytic action of SQ 29,852 would interfere with testing, making the animals less inclined to work. However, it is clear that SQ 29,852 is capable of improving performance in established learning tasks in the common marmoset.

Example 8

The following experiments were carried out to demonstrate the effectiveness of the ACE inhibitor SQ 29,852 in inhibiting loss of cognitive functions.

Adult male Sprauge-Dawley rats (Charles River, Wilmington, MA), age 25 weeks and weighing 350-400 g, were separately housed in stainless steel cages with continuous access to food and water, a 12-hour light-dark cycle, and constant room temperature of 22° to 24°C. All testing took place approximately 6 hours into the dark component of the light-dark cycle and was conducted in a dimly lighted soundproof room using a standard shuttle box device (Lehigh Valley Electronics #146-04), a plexiglass chamber (50 × 20 × 20 cm) divided by a center barrier 7 cm in height. The conditioned stimulus consisted of a 10-second

tone provided by a Sonlert mounted in the midpoint of the ceiling of the chamber. Floor current of 0.8 mA was delivered by a constant current shocker-scrambler (Lehigh Valley Electronics #133-33).

The day before the initiation of training each animal was allowed to explore the experimental chamber for 10 minutes without any tone or shock. Training was conducted for 15 days following the day of experimental chamber exploration. Each animal received 20 trials per day on a 30-second variable interval - schedule. No drug treatment was administered during the training period. Shock could be avoided by shuttling from one side of the center barrier to the other during the 10-second tone period. If an animal did not cross the center barrier during this period, the tone remained on and the floor shock was delivered until the animal escaped to the other half of the chamber. Animals which consistently remained on the center barrier were removed from the study. Automatic counters recorded the number of avoidance responses, escapes, and intertrial crossings, while a running time meter recorded the total shock duration for each animal.

Animals not meeting the admittance criterion of correct avoidance rsponding on at least 85 % of the trials for 4 out of the last 5 days of training were removed from the study. A total of 36 rats reaching the admittance criterion were tested for extinction of conditioned avoidance response (CAE) during 14 days. They were randomly assigned to SQ 29,852 (1 mg/kg, 10 mg/kg and 30 mg/kg) and saline control, with each test group at each dosage comprising 9 rats. All solutions were prepared fresh and administered i.p. (1 mg/ml volume) on the 2 days prior to testing and then 1 hour before it. Testing consisted of 20 trials per day identical to those previously described, except that no shock was administered if an animal failed to shuttle during the 10-second tone period. The tone was simply discontinued and the testing proceeded.

Two-way analysis of variance of the CAE data yielded an overall significant difference in the rate of shuttle extinction between treatment groups.

The present findings as seen from the accompanying Figure indicate that the ACE inhibitor, Q 29,852, possesses protective effects on memory of previously learned tasks while the saline control which does not have ACE inhibiting activity does not have protective effects against loss of memory of previously learned tasks.

It will also be appreciated that all of the above compounds and formulations may be employed in treating or delaying progression of Alzheimer's disease.

## Claims

1. Use of an angiotensin converting enzyme inhibitor which is a phosphonate substituted amino or imino acid cr salt thereof for the preparation of a pharmaceutical drug for inhibiting loss of cognitive function in a mammalian species over a prolonged period of time.

2. Use as defined in Claim 1 wherein said angiotensin converting enzyme inhibitor containing pharmaceutical drug is administered orally or parenterally.

3. Use as defined in Claim 1 wherein said angiotensin converting enzyme inhibitor is admixed with a pharmaceutically acceptable carrier therefor.

4. Use as defined in Claim 1 wherein the angiotensin converting enzyme inhibitor has the formula

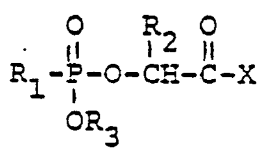

and pharmaceutically acceptable salts thereof wherein:

X is

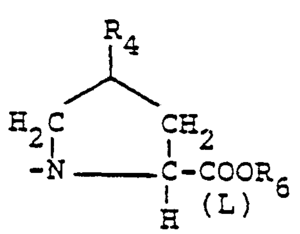 ,

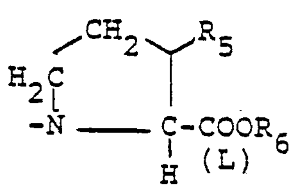 ,

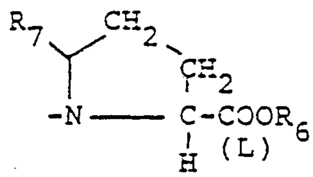 , 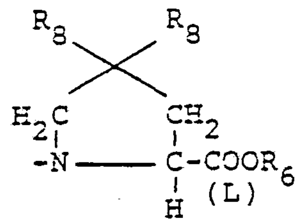 ,

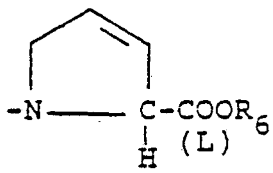 ,

$R_4$ is hydrogen, lower alkyl, halogen, keto, hydroxy, $-NH-\overset{O}{\overset{\|}{C}}-$lower alkyl, azido, amino, 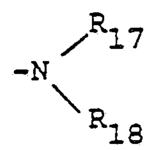 ,

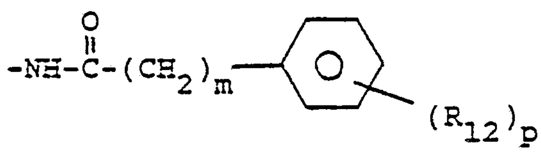 ,

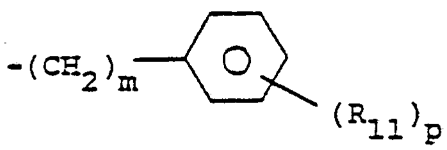 , 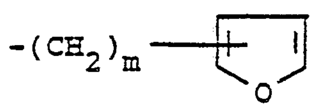 ,

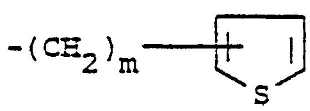 , 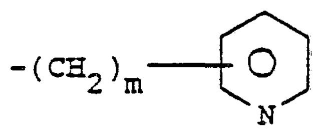 ,

a 1- or 2-naphthyl of the formula

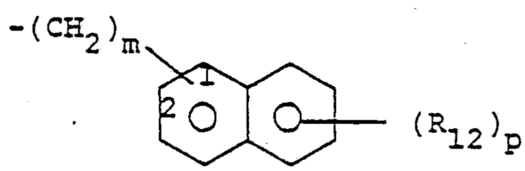 ,

$-(CH_2)_m$-cycloalkyl, 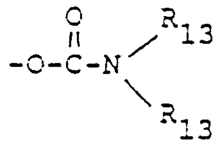 , -O-lower alkyl,

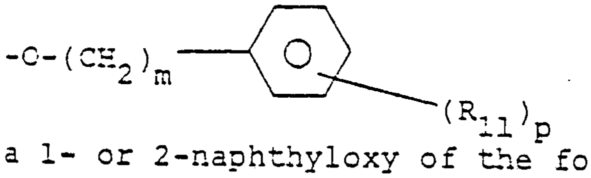 ,

a 1- or 2-naphthyloxy of the formula

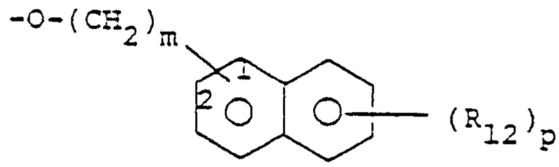 ,

-S-lower alkyl,

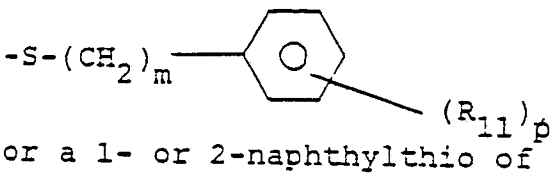 ,

or a 1- or 2-naphthylthio of the formula

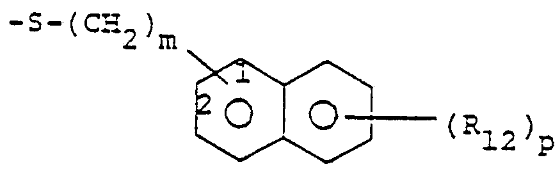 ,

$R_5$ is keto, halogen, 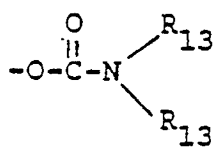 ,

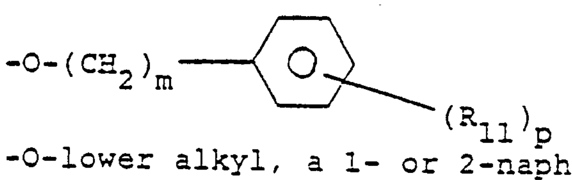 ,

-O-lower alkyl, a 1- or 2-naphthyloxy of the formula

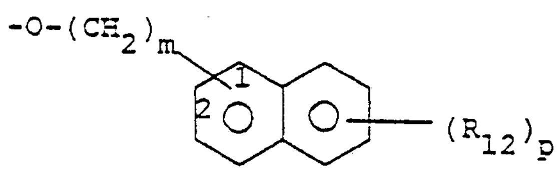 ,

-S-lower alkyl,

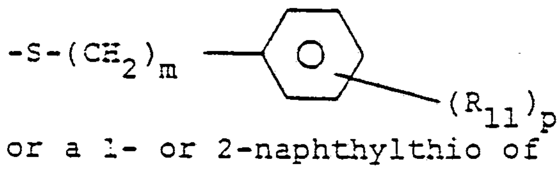 ,

or a 1- or 2-naphthylthio of the formula

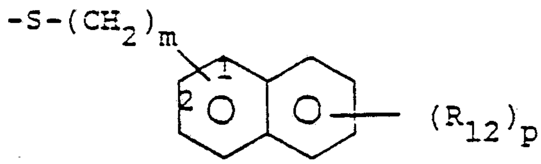 ,

$R_7$ is keto or

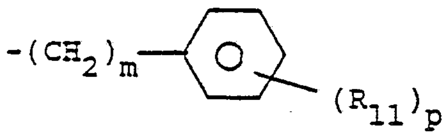 ,

each $R_8$ is the same or different and is halogen or $-Y-R_{14}$;

$R_{11}$ is hydrogen, lower alkyl of 1 to 4 carbons, lower alkoxy of 1 to 4 carbons, lower alkylthio of 1 to 4 carbons, chloro, bromo, fluoro, trifluoromethyl, hydroxy, phenyl, phenoxy, phenylthio, or phenylmethyl;

$R_{12}$ is hydrogen, lower alkyl of 1 to 4 carbons, lower alkoxy of 1 to 4 carbons, lower alkylthio of 1 to 4 carbons, chloro, bromo, fluoro, trifluoromethyl or hydroxy;

m is zero, one, two or three;

p is one, two or three provided that p is more than one only if $R_{11}$ or $R_{12}$ is hydrogen, methyl, methoxy, chloro or fluoro;

$R_{13}$ is hydrogen or lower alkyl of 1 to 4 carbons;

Y is oxygen or sulfur.

$R_{14}$ is lower alkyl of 1 to 4 carbons,

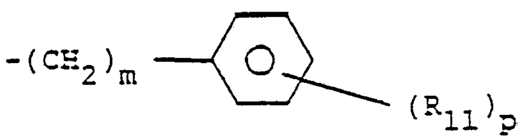 ,

or the $R_{14}$ groups join to complete an unsubstituted 5-or 6-membered ring or said ring in which one or more of the carbons has a lower alkyl of 1 to 4 carbons or a di(lower alkyl of 1 to 4 carbons) substituent;

$R_1$ is alkyl of 1 to 10 carbons, aminoalkyl, haloalkyl,

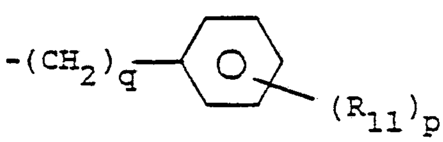 , $-(CH_2)_q$-cycloalkyl,

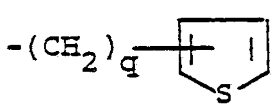 , 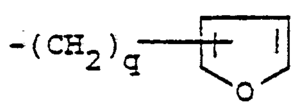 ,

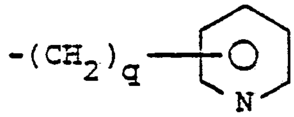 , or 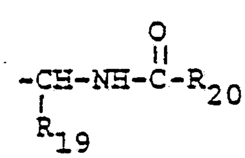

wherein q is zero or an integer from 1 to 7 and $R_{11}$ and p are as defined above;

$R_{19}$ and $R_{20}$ are independently selected from hydrogen, lower alkyl, halo substituted lower alkyl,

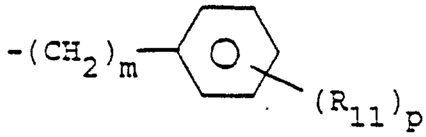 , $-(CH_2)_m$-cycloalkyl,

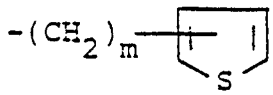 , 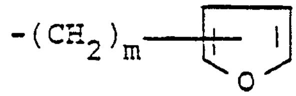 ,

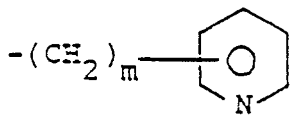 , or 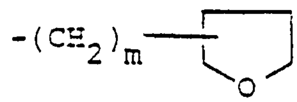

wherein m, $R_{11}$, and p are as defined above;

$R_2$ is hydrogen, lower alkyl, halo substituted lower alkyl,

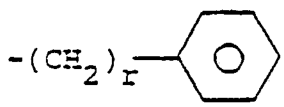 , 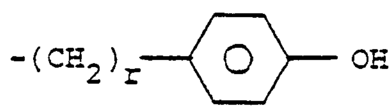 ,

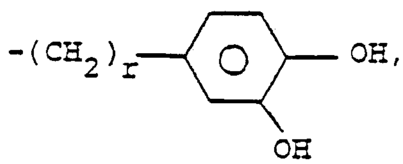 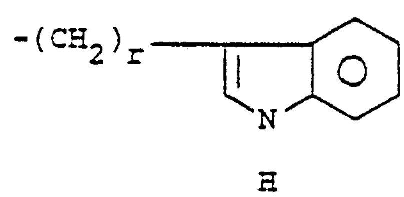 ,

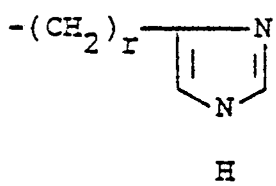 , $-(CH_2)_r-NH_2$, $-(CH_2)_r-SH$,

$-(CH_2)_r-S-$ lower alkyl, 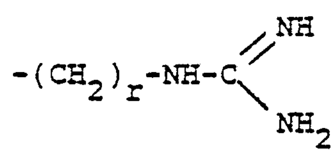 or

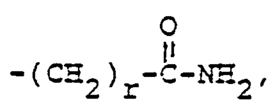

wherein r is an integer from 1 to 4;

$R_3$ and $R_6$ are independently selected form hydrogen, lower alkyl, benzyl, benzhydryl, alkali metal or

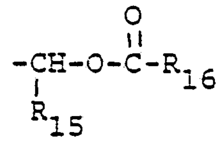

wherein $R_{15}$ is hydrogen, lower alkyl, cycloalkyl or phenyl, and $R_{16}$ is hydrogen, lower alkyl, lower alkoxy, phenyl, or $R_{15}$ and $R_{16}$ taken together are $-(CH_2)_2-$, $-(CH_2)_3-$, $-CH=CH-$, or

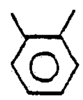 ;

$R_{17}$ is lower alkyl, benzyl, or phenethyl;

and

$R_{18}$ is hydrogen, lower alkyl, benzyl or phenethyl.

5. Use as defined in Claim 4 wherein in the compound employed $R_4$ is hydrogen, hydroxy, chloro, fluoro, lower alkyl of 1 to 4 carbons, cyclohexyl, amino, -O-lower alkyl wherein lower alkyl is of 1 to 4 carbons,

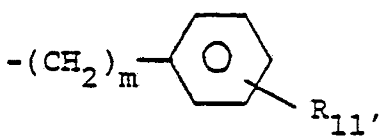 , 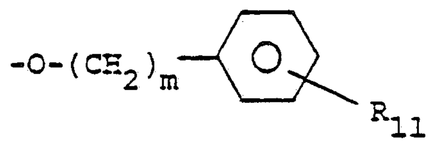 ,

1-or 2-naphthyloxy, -S-lower alkyl wherein lower alkyl is of 1 to 4 carbons,

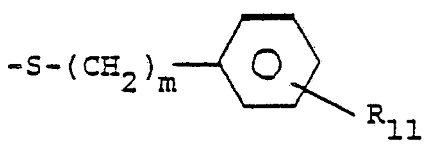 ,

or 1-or 2-naphthylthio;

$R_5$ is -O-lower alkyl wherein lower alkyl is of 1 to 4 carbons, -S-lower alkyl wherein lower alkyl is of 1 to 4 carbons,

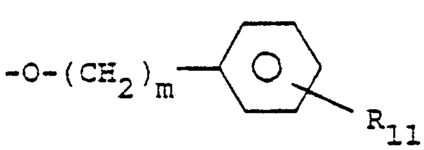 or 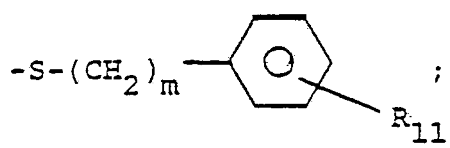 ;

$R_7$ is phenyl, 2-hydroxyphenyl, or 4-hydroxyphenyl; each $R_8$ is fluoro, chloro or $-Y-R_{14}$;

Y is oxygen or sulfur;

$R_{14}$ is lower alkyl of 1 to 4 carbons or the $R_{14}$ groups join to complete an unsubstituted 5-or 6-membered ring or said ring in which one or more of the carbons has a methyl or dimethyl substituent;

m is zero, one or two;

$R_{11}$ is hydrogen, methyl, methoxy, methylthio, chloro, bromo, fluoro or hydroxy;

$R_2$ is hydrogen, lower alkyl of 1 to 4 carbons, $CF_3$, or amino substituted lower alkyl of 1 to 4 carbons;

$R_1$ is alkyl of 1 to 10 carbons,

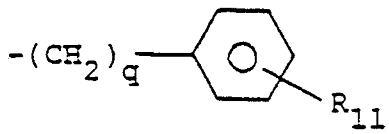,

$-(CH_2)_q$-cycloalkyl wherein cycloalkyl is of 5 or 6 carbons,

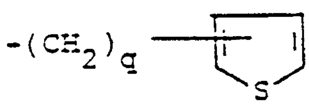, 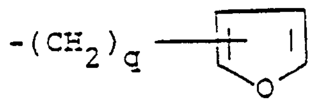,

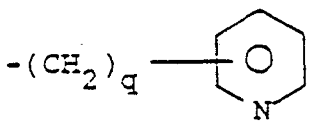, or 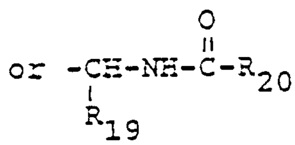

wherein q is zero or an integer from 1 to 4 and $R_{11}$ is as defined above;

$R_{19}$ and $R_{20}$ are independently selected from lower alkyl of 1 to 4 carbons or

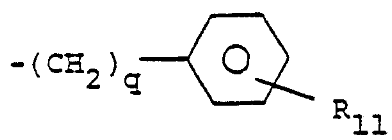

wherein q and $R_{11}$ are as defined above;

$R_3$ and $R_6$ are independently selected from hydrogen, alkali metal salt, lower alkyl of 1 to 4 carbons, or

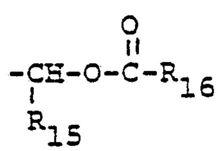;

$R_{15}$ is hydrogen, methyl or isopropyl; and

$R_{16}$ is lower alkyl of 1 to 4 carbons or phenyl.

6. Use as defined in Claim 5 wherein the compound employed X is

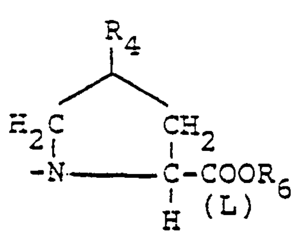, or 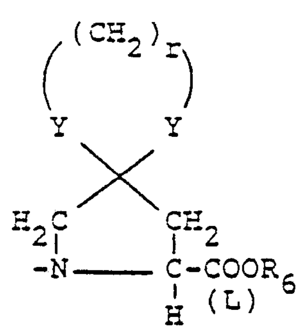,

$R_4$ is hydrogen, cyclohexyl, lower alkoxy of 1 to 4 carbons,

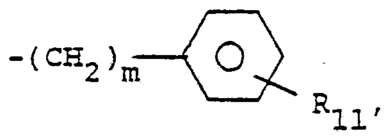 , 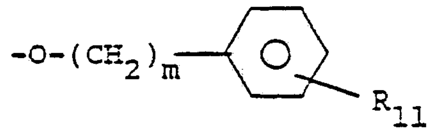 ,

or 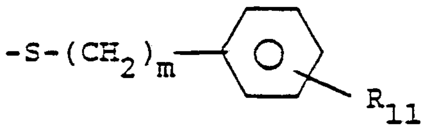 ;

m is zero, one or two;
$R_{11}$ is hydrogen, methyl, methoxy, methylthio, bromo, fluoro or hydroxy;
Y is oxygen or sulfur;
t is two or three; and
$R_6$ is hydrogen,

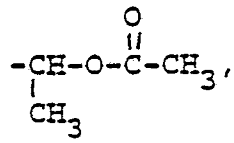 ,

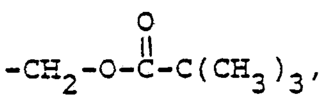 , 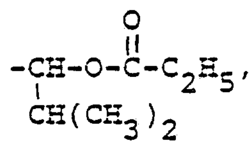 ,

or an alkali metal salt,
$R_1$ is

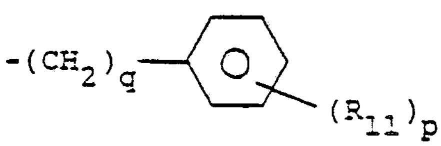 or 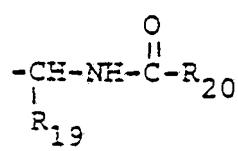 ;

$R_2$ is hydrogen or lower alkyl; and $R_3$ is hydrogen, alkali metal or phenylalkyl.

7. Use as defined in Claim 6 where in the compound employed $R_1$ is phenylalkyl or

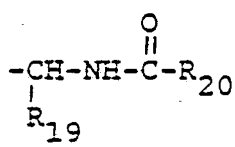

wherein $R_{19}$ is phenylalkyl and $R_{20}$ is phenyl;
$R_2$ is hydrogen, methyl, or $-(CH_2)_4-NH_2$; and
$R_3$ is hydrogen, phenylmethyl,

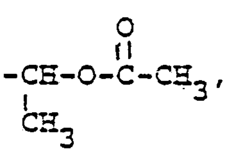 , 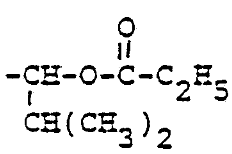 , 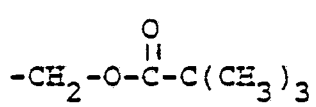 ,

or an alkali metal salt.

8. Use as defined in Claim 7 where in the compound employed $R_1$ is alkyl of 1 to 10 carbons.

9. Use as defined in Claim 8 where in the compound employed X is

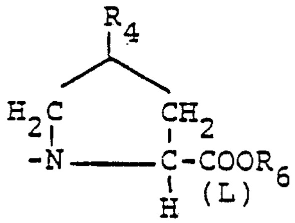

,

and $R_4$ is hydrogen.

10. Use as defined in Claim 6 where in the compound employed $R_1$ is phenylbutyl; $R_2$ is methyl; and $R_3$ and $R_6$ are an alkali metal salt.

11. Use as defined in Claim 6 where in the compound employed $R_1$ is

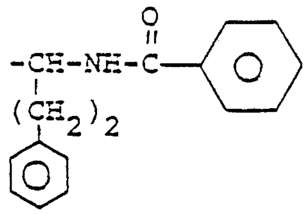

;

$R_2$ is methyl; and $R_3$ and $R_6$ are an alkali metal salt.

12. Use as defined in Claim 9 where in the compound employed $R_1$ is

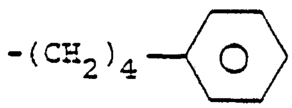

;

$R_2$ is methyl; $R_3$ is ethyl; and $R_6$ is an alkali metal salt.

13. Use as defined in Claim 1 wherein said angiotensin converting enzyme inhibitor is (S)-1-[6-amino-2-[[hydroxy(4-phenylbutyl phosphinyl]oxy]-1-oxohexyl]-L-proline (SQ 29,852).

14. Use as defined in Claim 1 wherein said angiotensin converting enzyme inhibitor containing pharmaceutical drug is to be administered in the form of tablets, capsules or by injection.

15. Use as defined in Claim 1 wherein said angiotensin converting enzyme inhibitor is (S)-1-[6-amino-2-[[hydroxy(4-phenylbutyl)phosphinyl]oxy]-1-oxohexyl-L-proline and the pharmaceutical drug containing the inhibitor is to be administered systemically in an amount of from about 0.1 to about 500 mg/l or 4 times a day.

16. Use of an angiotensin converting enzyme inhibitor which is a phosphonate substituted amino or imino acid or salt thereof for the preparation of a pharmaceutical drug for treating or delaying progression of Alzheimer's disease in a mammalian species.

17. Use according to any of Claims 1 to 15 wherein said angiotensin converting enzyme inhibitor containing drug is to be administered over a prolonged period of treatment to inhibit loss of cognitive function during such period of treatment.

18. Use as defined in Claim 17 wherein the angiotensin converting enzyme inhibitor is (S)-1-[6-amino-2-[[hydroxy(4-phenylbutyl)phosphinyl]oxy]-1-oxohexyl-L-proline.

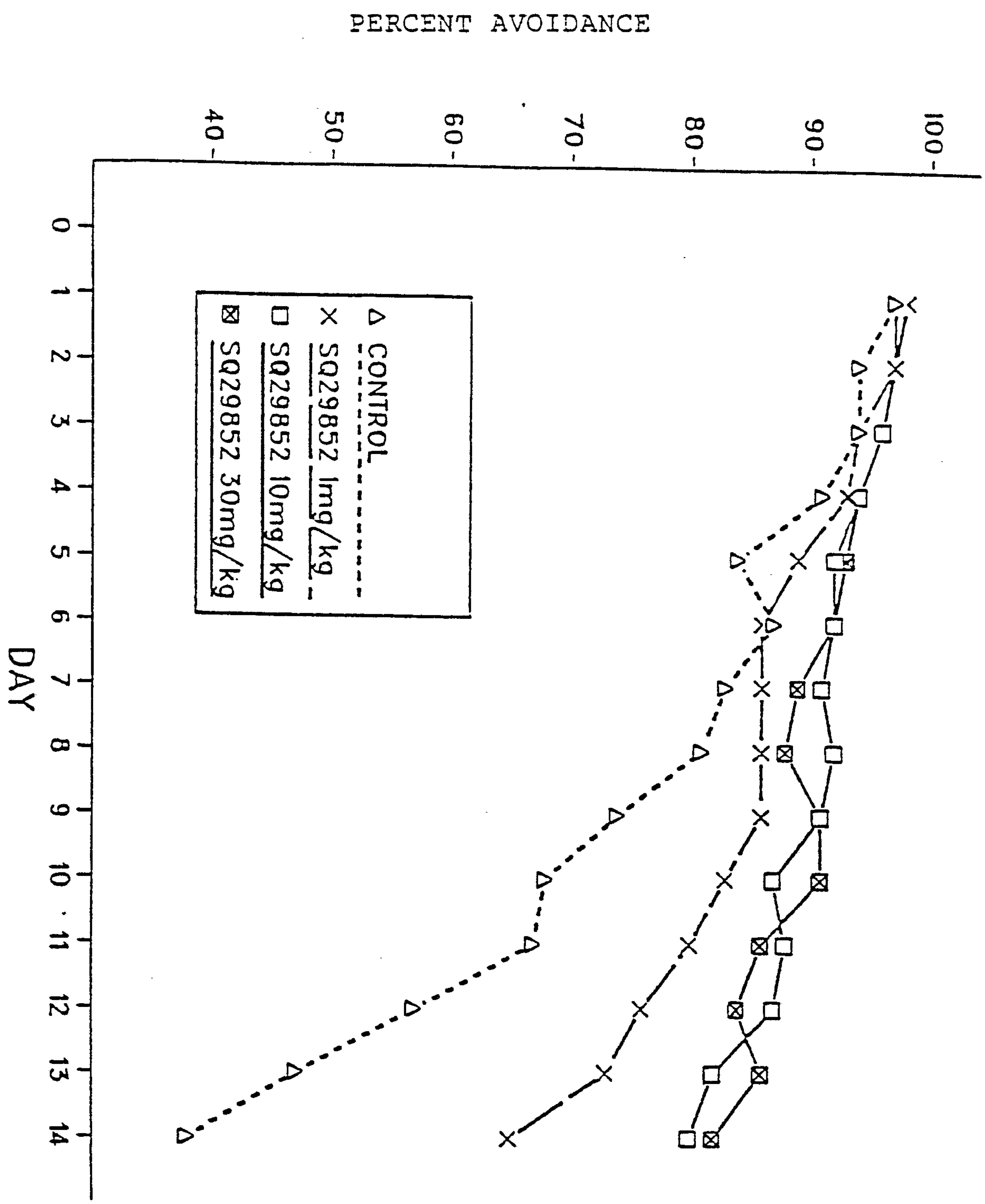
PERCENT AVOIDANCE
100-
90-
80-
70-
60-
50-
40-
DAY
0
1
2
3
4
5
6
7
8
9
10
11
12
13
14
CONTROL
SQ29852 1mg/kg
SQ29852 10mg/kg
SQ29852 30mg/kg